(19) Europäisches Patentamt
European Patent Office
Office européen des brevets

(11) Veröffentlichungsnummer: **0 058 885**
**B1**

(12) **EUROPÄISCHE PATENTSCHRIFT**

(45) Veröffentlichungstag der Patentschrift: **07.03.84**

(51) Int. Cl.³: **C 07 D 253/06,** A 01 N 43/64 // C07C103/19, C07C121/48

(21) Anmeldenummer: **82100997.4**

(22) Anmeldetag: **11.02.82**

(54) **Herbizid wirksames 4-Amino-6-(2-chlor-1-methyl-cyclopropyl)-3-methylthio-1,2,4-triazin-5-on.**

(30) Priorität: **23.02.81 DE 3106707**

(43) Veröffentlichungstag der Anmeldung: **01.09.82 Patentblatt 82/35**

(45) Bekanntmachung des Hinweises auf die Patenterteilung: **07.03.84 Patentblatt 84/10**

(84) Benannte Vertragsstaaten: **BE CH DE FR GB IT LI NL**

(56) Entgegenhaltungen:
**DE - A - 2 726 016**
**DE - A - 2 732 797**
**DE - A - 2 733 180**
**GB - A - 1 182 801**
**GB - A - 1 182 802**

(73) Patentinhaber: **Degussa Aktiengesellschaft, Weissfrauenstrasse 9, D-6000 Frankfurt am Main 1 (DE)**

(72) Erfinder: **Schwarze, Werner, Dr., Leerbachstrasse 17, D-6000 Frankfurt (Main) (DE)**
Erfinder: **Kleemann, Axel, Dr., Greifenhagenstrasse 9, D-6450 Hanau 9 (DE)**
Erfinder: **Leuchtenberger, Wolfgang, Dr., Geschwister-Scholl-Strasse 1, D-6454 Bruchköbel 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 058 885 B1**

# Herbizid wirksames 4-Amino-6-(2-chlor-1-methyl-cyclopropyl)-3-methylthio-1,2,4-triazin-5-on

Die Erfindung betrifft die neue Verbindung 4-Amino-6-(2-chlor-1-methyl-cyclopropyl)-3-methylthio1,2,4-triazin-5-on, Verfahren zu ihrer Herstellung, herbizide Mittel, welche als wirksame Komponente diese Verbindung enthalten, sowie ihre Verwendung oder die sie enthaltenden Mittel zur Bekämpfung unerwünschten Pflanzenwachstums.

Das neue Derivat entspricht der Formel I:

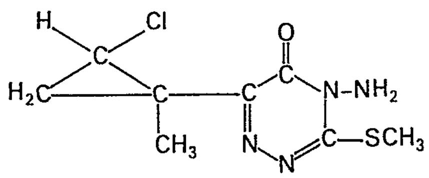

(I)

Triazinonderivate der allgemeinen Formel

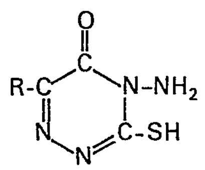

können nach der Methode von Dornow [Ber. 97, p. 2173-79 (1964)] hergestellt werden, indem man die entsprechenden Ketocarbonsäuren mit Thiocarbohydrazid kondensiert.

So ist z.B. bereits das 4-Amino-6-(2,2-dichlor-1-methyl-cyclopropyl)-3--methylthio-1,2,4-triazinon-5-on bekannt geworden. Diese Verbindung zeigt selbst bei geringen Aufwandmengen (500 g/ha) eine hervorragende herbizide Wirkung auf unerwünschten Pflanzenwuchs, unter deutlicher Schonung einer Reihe von Nutzpflanzenkulturen. Demgegenüber war es durchaus überraschend, dass der Wirkstoff nach der Erfindung, selbst wenn er in geringeren Mengen (400 g/ha) eingesetzt wird, eine Wirkung zeigt, die der durch den Wirkstoff der Entgegengehaltenen zumindest ebenbürtig, in manchen Fällen jedoch überlegen ist, z.B. im Falle Lolium perenne bei der Vorauflauf-Anwendung. Weiterhin ist es nicht zu erwarten gewesen, dass die Nutzpflanzen Soja hispida und Secale cereale durch den Wirkstoff nach der Erfindung ebenfalls in der Vorauflaufanwendung besser toleriert werden. Während z.B. im letzten Falle bei Anwendung der vorbekannten Verbindung eine Schädigung nach der Bonitierung 4 eintritt, ist bei der erfindungsgemässen Verbindung nur 2 gegeben. Hieraus ergibt sich eine höchst wünschenswerte Bereicherung der für die Unkrautbekämpfung bereitgestellten Wirkstoffe. Für die Herstellung der Verbindung I müsste für die Reaktion die Ketosäure

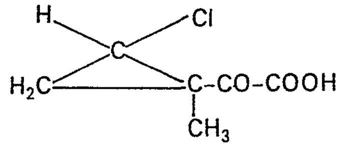

zur Verfügung stehen. Diese Verbindung ist jedoch unbekannt und kann auch nicht nach den üblichen Verfahren hergestellt werden.

Es wurde nun gefunden, dass die Kondensation zum gewünschten 4-Amino-6-(2-chlor-1-methyl-cyclopropyl)-3-methylthio-1,2,4-triazin-5-on in guter Ausbeute gelingt, wenn man vom 2-Chlor-1-methylcyclopropylglyoxylsäure-t-butylamid ausgeht und dieses mit Thiocarbohydrazid in Gegenwart von Mineralsäuren bei erhöhter Temperatur umsetzt. Bevorzugte Lösungsmittel sind Gemische von Alkoholen mit Wasser, bevorzugte Mineralsäure ist die Salzsäure. Das benötigte t-Butylamid kann nach dem in der DE-AS 27 33 181 offenbarten Verfahren aus dem entsprechenden Acylcyanid hergestellt werden.

Das für die Synthese des erwünschten t-Butylamids als Ausgangsstoff dienende Acylcyanid ist aus dem entsprechenden Acylchlorid erhältlich. Zum Beispiel kann es nach dem Verfahren gemäss DE-PS 27 08 183 durch Umsetzung mit CuCN bei Temperaturen zwischen 50 und 180°C in einem Gemisch aus 1 bis 10 Äquivalenten eines inerten Carbonsäurenitrils und etwa 0,5 bis 20 Gew.-Teilen eines inerten organischen Lösungsmittels, zum Beispiel Dioxan, erhalten werden. Nach der DE-PS 27 08 182 kann diese Herstellung aber auch so erfolgen, dass man ein Gemisch aus etwa 0,1 bis 5 Äquivalenten eines Alkalimetallcyanids und etwa 0,05 bis 2 Äquivalenten eines Kupfer(I)-Salzes verwendet und ebenfalls in Gegenwart eines inerten Carbonsäurenitrils arbeitet.

Das erfindungsgemässe 4-Amino-6-(2-chlor-1-methyl-cyclopropyl)-3-methylthio-1,2,4-triazin-5-on beeinflusst das Pflanzenwachstum und zeigt insbesondere ausgezeichnete herbizide Eigenschaften. Es dient vor allem der Unkrautbekämpfung, kann aber auch aufgrund seiner vorteilhaften austrocknenden und entblätternden Wirksamkeit als Erntehilfsmittel in Kulturen wie Baumwolle oder Kartoffeln eingesetzt werden.

Bei der Bekämpfung mono- und dikotyler Unkräuter zeigt diese Verbindung selbst bei geringen Aufwandmengen eine hervorragende herbizide Wirkung auf unerwünschten Pflanzenwuchs unter deutlicher Schonung einer Reihe von Nutzpflanzenkulturen und ist darin strukturell ähnlichen bekannten 1,2,4-Triazin-5-on-Derivaten überraschend überlegen. Es werden dabei auch schwer bekämpfbare Unkrautarten erfasst.

Das erfindungsgemässe 4-Amino-6-(2-chlor-1-methyl-cyclopropyl)-3-methylthio-1,2,4-triazin-5-on besitzt sehr gute Eigenschaften gegen Gramineen, wie Hirse oder hirseartige Pflanzen oder Ackerfuchsschwanz (Alpecurus sp.).

Die allgemeine Wirksamkeit der Triazinone ist bekannt, zum Beispiel die der Verbindung 4-Amino-6-t-butyl-3-methylthio-1,2,4-triazin-5-on. Von dieser unterscheidet sich die Wirksamkeit der neuen Verbindung vor allem im Vorauflaufversuch bei mittleren Konzentrationen dadurch, dass sie in einigen Nutzpflanzenkulturen, zum Beispiel in Mais, Soja, Roggen, Gerste und Hafer, sehr viel selektiver wirkt.

Die Aufbringung der erfindungsgemässen Verbindung kann in den üblichen Formulierungen mit gleich gutem Erfolg sowohl nach dem Auflaufen (postemergent) als auch insbesondere vor dem Auflaufen (preemergent) der Pflanzen erfolgen. Die Aufwandmenge für die erfindungsgemässe Verbindung können abhängig von Einsatzzweck, Einsatzort, Kultur, Schadpflanzenarten und -besatz, klimatischen Verhältnissen usw. in breiten Grenzen schwanken und sind überraschend gering. In leichten Böden wirkt das 4-Amino-6-(2-chlor-1-methyl-cyclopropyl)-3-methylthio-1,2,4-triazin-5-on im allgemeinen in Aufwandmengen schon ab 0,1 kg pro Hektar und wird vorzugsweise in Aufwandmengen von 0,2 bis 0,8 kg pro Hektar eingesetzt; in schweren humusreichen und adsorptiven Böden sind höhere Aufwandmengen anzuwenden. Bei Aufwandmengen von 1,0 kg pro Hektar und mehr tritt die totalherbizide Wirkung dieser Verbindung gegenüber der Selektivwirkung in den Vordergrund.

Die folgenden Beispiele veranschaulichen die Herstellungsverfahren für das 4-Amino-6-(2-chlor-1-methyl-cyclopropyl)-3-methylthio-1,2,4-triazin-5-on. Die Temperaturen sind darin in Celsiusgraden angegeben.

**Herstellung**

a) Synthese von 2-Chlor-1-methyl-cyclopropancarbonsäurecyanid

In einen 500 ml-Dreihalskolben mit Rührer, Thermometer und Rückflusskühler werden in einer Mischung von 50 ml Acetonitril und 100 ml Toluol 68,6 g Natriumcyanid (= 1,4 mol) und 12,6 g Kupfer(I)-Cyanid (= 0,14 mol) gegeben. Unter Rühren tropft man innerhalb von 10 Minuten 153 g 2-Chlor-1-methyl-cyclopropancarbonsäurechlorid (= 1 mol) hinzu. Man erhitzt unter Rückfluss. Nach 8 Stunden ist im IR-Spektrum keine Säurechlorid-Bande mehr festzustellen. Man destilliert und erhält bei $Kp_{16\ mbar}$ 74 bis 78°C das gewünschte Säurecyanid, Menge: 117,7 g entsprechend 82,02% d.Th..

b) Synthese von 2-Chlor-1-methyl-cyclopropylglyoxylsäure-t-butylamid

In einem 1 ltr.-Dreitubuskolben mit Rührer, Thermometer und Tropftrichter werden 121,4 g t-Butanol und 75 ml $CH_2Cl_2$ vorgelegt Bei 0°C gibt man 117,7 g 2-Chlor-1-methyl-cyclopropancarbonsäurecyanid hinzu. Innerhalb von 30 Minuten tropft man nun bei Temperaturen von − 10°C bis 0°C 120 g konz. $H_2SO_4$ hinzu, rührt anschliessend 1 Stunde bei 0°C, dann 3 Stunden bei 20°C. Nun kühlt man wieder herunter auf 0°C und tropft dann 16,3 ml Wasser hinzu, rührt noch 15 Minuten, fügt 200 ml $CH_2Cl_2$ dazu und neutralisiert bei Temperaturen von 15 bis 20°C mit 25%iger Natronlauge. Die organische Phase wird nun abgetrennt, getrocknet und eingedampft. Man erhält ein rötliches Öl, 167 g = 94% d.Th. Reaktionsprodukt.

Analyse: $C_{10}H_{16}ClNO_2$ (mol-Gew. 217,5)

| | | | | |
|---|---|---|---|---|
| ber. | C 55,2 | H 7,4 | N 6,4 | Cl 16,3 |
| gef. | 55,1 | H 7,2 | N 6,2 | Cl 16,1 |

c) Synthese von 4-Amino-3-thiono-6-(2-chlor-1-methyl-cyclopropyl)-1,2,4-triazin-5-on

108,75 g 2-Chlor-1-methyl-cyclopropylglyoxylsäure-t-butylamid (= 1/2 mol) und 58,3 g Thiocarbohydrazid (= 0,55 mol) gibt man in eine Mischung von 600 ml Äthanol + 55 g konz. Salzsäure. Man erhitzt zum Sieden. Nach 6 Stunden kühlt man ab, dabei erstarrt die Lösung unter Abscheidung des Reaktionsproduktes. Es wird auf 0°C heruntergekühlt und dann abgenutscht und mit eiskaltem 80%igem Äthanol und Wasser gewaschen. Trocknen bei 60°C/16 mbar. Es verbleiben 94,6 g gelbe Kristalle, F. 162–163°C.
Ausbeute 81,2% d.Th.

Analyse: $C_7H_9ClN_4OS$ (mol-Gew. 232,5)

| | | | | | |
|---|---|---|---|---|---|
| ber. | C 36,1 | H 3,9 | Cl 15,3 | N 24,1 | S 13,8 |
| gef. | 36,3 | H 3,9 | Cl 14,9 | N 23,8 | S 14 |

d) Synthese von 4-Amino-3-methylthio-6-(2-chlor-1-methyl-cyclopropyl)-1,2,4-triazin-5-on

58,1 g 4-Amino-3-thiono-6-(2-chlor-1-methyl-cyclopropyl)-1,2,4-triazin-5-on (= 0,25 mol) löst man in 260 ml n/l-NaOH und filtriert. Die Lösung wird auf 0°C abgekühlt. Nun tropft man innerhalb von 45 Minuten 35,5 g $CH_3J$ (= 0,25 mol) und anschliessend nochmals 3,55 g $CH_3J$ hinzu. Nach etwa 30 Minuten beginnt die Kristallabscheidung. Man rührt etwa 6 Stunden, dann ist die Lösung neutral.

Man saugt ab, wäscht mit Wasser und trocknet. Hellgraue Kristalle, F.76–78°C. Menge 51,8 g entsprechend 84,1% d.Th.

Analyse: $C_8H_{11}ClN_4OS$ (mol-Gew. 246,5)

| | | | | | |
|---|---|---|---|---|---|
| ber. | C 38,9 | H 4,46 | Cl 14,4 | N 22,7 | S 12,98 |
| gef. | 38,9 | H 4,50 | Cl 14,4 | N 22,2 | S 12,7 |

Das Produkt kann aus Benzin-Petroläther umkristallisiert werden und liefert weisse Kristalle vom F. 78–78,5°C.

Die herbizide Wirkung des 4-Amino-6-(2-chlor-1-methyl-cyclopropyl)-3-methylthio-1,2,4-triazin-5-on wurde durch folgende Versuche gezeigt:

1. Herbizide Wirkung bei Applikation der Wirkstoffe vor dem Auflaufen der Pflanzen (preemergent).

Im Gewächshaus wurden Testpflanzen in Kästen eingesät, und zwar Kulturpflanzen und

mono- und dicotyledone Unkräuter. Nach Einsaat wurden der Wirkstoff 4-Amino-6-(2-chlor-1-methyl-cyclopropyl)-3-methylthio-1,2,4-triazin-5-on sowie ein handelsübliches Triazinonderivat als wässrig-äthanolische Dispersion aufgesprüht. Die Auswertung der erzielten Wirkung an den aufgelaufenen Pflanzen erfolgte 28 Tage nach der Applikation. Während des Versuches wurden die Pflanzen gleichmässig feucht gehalten.

Die Bonitierung erfolgte nach dem 6-er-Index:

1 Pflanzen ungeschädigt
2 Mässiger Wuchs
3 Pflanze kümmert
4 Leichter Schaden
5 Starker Schaden
6 Totalschaden

2. Herbizide Wirkung bei Applikation der Wirkstoffe nach dem Auflaufen der Pflanzen (postemergent)

In einem Gewächshaus wurden in Kästen gesäte und aufgeschossene Kulturpflanzen und Unkräuter im 4- bis 6-Blattstadium mit einer wässrig-äthanolischen Suspension gespritzt. Die Auswertung erfolgte 28 Tage nach der Behandlung. Die Bonitierung erfolgte wie unter 1. angegeben.

Geprüfte Verbindungen:

A: 4-Amino-6-t-butyl-3-methylthio-1,2,4-triazin-5-on (vorbekannt)
B: 4-Amino-6-(2-chlor-1-methylcyclopropyl)-3-methylthio-1,2,4-triazin-5-on (gemäss Erfindung).

| Testpflanze | Vorauflauf 400 g/ha | | Nachauflauf 400 g/ha | |
|---|---|---|---|---|
| | A | B | A | B |
| Beta vulgaris | 6 | 6 | 6 | 6 |
| Soja hispida | 6 | 1-2 | 5 | 6 |
| Triticum aestivum | 6 | 2 | 5 | 4 |
| Secale cereale | 6 | 2 | 6 | 5 |
| Hordeum vulgare | 4 | 2 | 3 | 4 |
| Avena sativa | 6 | 2 | 6 | 5 |
| Lolium perenne | 6 | 5 | 6 | 5 |
| Stellaria media | 6 | 6 | 6 | 6 |
| Polygonum persicaria | 6 | 6 | 6 | 6 |
| Galium aparine | 2 | 4 | 4 | 5 |
| Echinochloa crus galli | 6 | 6 | 6 | 6 |
| Chenopodium album | 6 | 6 | 6 | 6 |
| Avena fatua | 6 | 6 | 6 | 6 |
| Amaranthus retroflexus | 6 | 6 | 6 | 6 |

Zur Herstellung von herbiziden Mitteln wird der Wirkstoff mit geeigneten Trägerstoffen und/oder Verteilungsmitteln kombiniert. Dies erfolgt in an sich bekannter Weise durch inniges Vermischen und Vermahlen des Wirkstoffs mit geeigneten Trägerstoffen, gegebenenfalls unter Zusatz von gegenüber den Wirkstoffen inerten Dispersions- oder Lösungsmitteln. Der Wirkstoff kann als Stäubemittel, Streumittel, Granulate, Umhüllungsgranulate, Imprägnierungsgranulate, Homogengranulate, Spritzpulver (wettable powder), Pasten, Emulsionen, Lösungen oder Aerosile verwendet werden.

Zur Herstellung fester Aufarbeitungsformen (Stäubemittel, Streumittel, Granulate) wird der Wirkstoff mit festen Trägerstoffen vermischt. Die Korngrösse der Trägerstoffe beträgt für Stäubemittel zweckmässig bis ca. 0,1 mm, für Streumittel ca. 0,075 bis 0,2 mm und für Granulate 0,2 mm oder mehr. Die Wirkstoffkonzentrationen in den festen Aufarbeitungsformen betragen in der Regel 0,5 bis 80%. Diesen Gemischen können ferner den Wirkstoff stabilisierende Zusätze und/oder nichtionische anionaktive und kationaktive Stoffe zugegeben werden, die beispielsweise die Haftfestigkeit des Wirkstoffs auf Pflanzen und Pflanzenteile verbessern (Haft- und Klebemittel) und/oder eine bessere Benetzbarkeit (Netzmittel) sowie Dispergierbarkeit (Dispergatoren) gewährleisten.

In Wasser dispergierbare Wirkstoffkonzentrate, Spritzpulver (wettabele powder), Pasten und Emulsionskonzentrate, stellen Mittel dar, die mit Wasser auf jede gewünschte Konzentration verdünnt werden können. Sie bestehen aus Wirkstoff, Trägerstoff, gegebenenfalls den Wirkstoff stabilisierenden Zusätzen, oberflächenaktiven Substanzen und Antischaummitteln und gegebenenfalls Lösungsmitteln. Die Wirkstoffkonzentration in diesen Mitteln beträgt 5 bis 80%. Die Spritzpulver (wettable powder) und Pasten werden erhalten, indem man den Wirkstoff mit Dispergiermitteln und pulverförmigen Trägerstoffen in geeigneten Vorrichtungen bis zur Homogenität vermischt und vermahlt. In manchen Fällen ist es vorteilhaft, Mischungen verschiedener Trägerstoffe zu verwenden. Als Antischaummittel kommen zum Beispiel Silicone in Frage. Der Wirkstoff wird mit den oben aufgeführten Zusätzen so vermischt, vermahlen, gesiebt und passiert, dass bei Spritzpulvern der feste Anteil eine Korngrösse von 0,02 bis 0,04 und bei Pasten von 0,003 mm nicht überschreitet. Zur Herstellung von Emulsionskonzentraten und Pasten werden Dispergiermittel, organische Lösungsmittel und Wasser verwendet. Die Lösungsmittel müssen praktisch geruchlos, nicht phytotoxisch, dem Wirkstoff gegenüber inert und nicht leicht brennbar sein.

Ferner können die erfindungsgemässen Mittel in Form von Lösungen angewendet werden. Hierzu wird der Wirkstoff in geeigneten organischen Lösungsmitteln, Lösungsmittelgemischen oder Wasser gelöst. Die Lösungen sollen den Wirkstoff in einem Konzentrationsbereich von 1 bis 20% enthalten.

Granulat

Zur Herstellung eines 5%igen Granulates werden die folgenden Stoffe verwendet:

5 Teile 4-Amino-6-
(2-chlor-1-methyl-cyclopropyl)-3-methylthio-1,2,4-triazin-5-on
0,25 Teile Epichlorhydrin,
0,25 Teile Cetylpolyglykoläther mit 8 mol Äthylenoxid,
3,50 Teile Polyglykol («Carbowax»®),
91 Teile Kaolin (Korngrösse 0,3 bis 0,8 mm).

Die Aktivsubstanz wird mit Epichlorhydrin vermischt und mit 6 Teilen Aceton gelöst, hierauf wird Polyglykol und Cetylpolyglykoläther zugesetzt. Die so erhaltene Lösung wird auf Kaolin aufgesprüht und anschliessend das Aceton im Vakuum verdampft.

Spritzpulver

Zur Herstellung eines a) 50%igen, b) 25%igen und c) 10%igen Spitzpulvers werden folgende Bestandteile verwendet:

a) 50 Teile 4-Amino-6-(2-chlor-1-methyl-cyclopropyl)-3-methylthio-1,2,4-triazin-5-on
5 Teile Natriumdibutylnaphthylsulfonat,
3 Teile Naphthalinsulfonsäuren-Phenolsulfonsäuren-Formaldehyd-Kondensat 3:2:1,
20 Teile Kaolin,
22 Teile Champagne-Kreide;
b) 25 Teile des obengenannten Wirkstoffes
5 Teile Oleylmethyltaurid-Na-Salz,
2,5 Teile Naphthalinsulfonsäure-Formaldehyd-Kondensat,
0,5 Teile Carboxymethylcellulose,
5 Teile neutrales Kalium-Aluminiumsilikat,
62 Teile Kaolin;
c) 10 Teile des obengenannten Wirkstoffes
3 Teile Gemisch der Natriumsalze von gesättigten Fettalkoholsulfaten,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnen mit Wasser Suspensionen jeder gewünschten Wirkstoffkonzentration erhalten werden. Derartige Suspensionen können zur Bekämpfung von Unkräutern und Ungräsern in Baumwollpflanzungen verwendet werden.

Paste

Zur Herstellung einer 45%igen Paste werden folgende Stoffe verwendet:

45 Teile 4-Amino-6-(2-chlor-1-methyl-cyclopropyl)-3-methylthio-1,2,4-triazin-5-on
5 Teile Natriumaluminiumsilikat,
14 Teile Cetylpolyglykoläther mit 8 mol Äthylenoxid,
1 Teil Cetylpolyglykoläther mit 5 mol Äthylenoxid,
2 Teile Spindelöl,
10 Teile Polyglykol («Carbowax»®),
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 10%igen Emulsionskonzentrates werden

10 Teile 4-Amino-6-(2-chlor-1-methyl-cyclopropyl)-3-methylthio-1,2,4-triazin-5-on
15 Teile Cetylpolyglykoläther mit 8 mol Äthylenoxid,
75 Teile Isophoron (3,5,5-Trimethylcyclohex-2-en-1-on)

miteinander vermischt. Dieses Konzentrat kann mit Wasser zu Emulsionen auf geeignete Konzentrationen verdünnt werden. Solche Emulsionen eignen sich zur Bekämpfung von Unkräutern in Kulturpflanzen, wie zum Beispiel Sojabohnen und Kartoffeln etc.

**Patentansprüche**

1) 4-Amino-6-(2-chlor-1-methyl-cyclopropyl)-3-methylthio-1,2,4-triazin-5-on.

2) Herbizides Mittel, dadurch gekennzeichnet, dass es als Wirkstoff die Verbindung nach Anspruch 1 neben üblichen Hilfsstoffen enthält.

3) Verwendung der Verbindung nach Anspruch 1 oder der sie enthaltenden herbiziden Mittel zur Bekämpfung von Unkräutern.

4) Verfahren zur Herstellung der Verbindung nach Anspruch 1, dadurch gekennzeichnet, dass man 2-Chlor-1-methyl-cyclopropancarbonsäure über Säurechlorid und Säurecyanid mit t-Butanol oder Isobutylen in Schwefelsäure zum 2-Chlor-1-methyl-cyclopropylglyoxylsäure-t-butylamid umsetzt, dieses mit Thiocarbohydrazid in Gegenwart von mindestens molaren Mengen einer Mineralsäure zum 4-Amino-6-(2-chlor-1-methyl-cyclopropyl)-3-thiono-1,2,4-triazin-5-on kondensiert und anschliessend das Reaktionsprodukt in an sich bekannter Weise methyliert.

**Claims**

1. 4-amino-6-(2-chloro-1-methyl-cyclopropyl)-3-methylthio-1,2,4-triazin-5-one.

2. A herbicide, characterised in that it contains as active substance the compound according to claim 1 in addition to conventional auxiliaries.

3. The use of the compound according to claim 1 or of the herbicides containing said compound for weed control.

4. A process for the production fo the compound according to claim 1, characterised in that 2-chloro-1-methyl-cyclopropane carboxylic acid is reacted via acid chloride and acid cyanide with t-butanol or isobutylene in sulphuric acid to produce 2-chloro-1-methyl-cyclopropylglyoxylic acid-t-butylamide, this is condensed with thiocarbohydrazide in the presence of at least molar quantities of a mineral acid to produce 4-amino-6-(2-chloro-1-methyl-cyclopropyl)-3-thiono-1,2,4-triazin-5-one and the reaction product is then methylated in a known manner.

## Revendications

1. 4-Amino-6-(2-chloro-1-méthyl-cyclopropyl)-3-méthylthio-1,2,4-triazin-5-one.

2. Agent herbicide caractérisé en ce que comme matière active, il contient le composé de la revendication 1, ainsi que les matières auxiliaires habituelles.

3. Utilisation du composé de la revendication ou de l'agent herbicide qui le contient, pour lutter contre les mauvaises herbes.

4. Procédé de préparation du composé selon la revendication 1, caractérisé en ce que l'on fait réagir l'acide 2-chloro-1-méthyl-cyclopropane-carboxylique, en passant par le chlorure d'acide et le cyanure d'acide, avec le t-butanol ou l'isobutylène, dans l'acide sulfurique, pour obtenir le t-butylamide de l'acide 2-chloro-1-méthyl-cyclopropyl-glyoxylique, que l'on condense celui-ci avec le thiocarbohydrazide, en présence d'une proportion au moins molaire d'un acide minéral, en 4-amino-6-(2-chloro-1-méthyl-cyclopropyl)-3-thiono-1,2,4-triazine-5-one, et qu'ensuite on procède à la méthylation du produit réactionnel, d'une matière connue.